(19) **Europäisches Patentamt European Patent Office Office européen des brevets**

(11) **EP 2 815 808 A1**

(12) **EUROPEAN PATENT APPLICATION**
published in accordance with Art. 153(4) EPC

(43) Date of publication:
**24.12.2014 Bulletin 2014/52**

(21) Application number: **12877453.6**

(22) Date of filing: **09.07.2012**

(51) Int Cl.:
***B01F 3/00*** *(2006.01)*    ***B01F 11/02*** *(2006.01)*
***A61K 8/06*** *(2006.01)*

(86) International application number:
**PCT/RU2012/000552**

(87) International publication number:
**WO 2013/176565 (28.11.2013 Gazette 2013/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.05.2012 RU 2012120584**

(71) Applicant: **Cavitanica Ltd.**
**420107, Kazan (RU)**

(72) Inventors:
• **GETALOV, Andrey Aleksandrovich**
**Moscow 109548 (RU)**
• **DEDYUKHIN, Evgeny Evgen'evich**
**Kazan 420043 (RU)**
• **GINIYATULLIN, Marat Munirovich**
**Kazan 420043 (RU)**
• **SIROTKIN, Aleksandr Semenovich**
**Kazan 420097 (RU)**

(74) Representative: **Benatov, Emil Gabriel et al**
**Dr. Emil Benatov & Partners**
**Asen Peykov Str. No. 6**
**1113 Sofia (BG)**

(54) **METHOD FOR THE SIMULTANEOUS ULTRASONIC CAVITATION TREATMENT OF CONTAINERS OF LIQUID MEDIA**

(57) The invention relates to the field of cavitation treatment of liquid media and also of media in which the specific content of water or another liquid phase exceeds 30-35 % of the total mass. The method consists in placing containers of liquid media in a working fluid in a rectangular bath. The material of the liquid media-filled containers has a specific acoustic resistance equal to or approximating the specific acoustic resistance of the working fluid. A standing acoustic wave is created in the working fluid from all walls and the base of the bath, which are designed as flexible membranes having their own first harmonic resonance frequency. The length (a) and width (b) of the bath are chosen as multiples of one quarter of the length of the wave created in the working fluid by the side walls of the bath. The height (h) of the working fluid level is chosen as a multiple of one quarter of the length of the wave created by the base of the bath. The method enables the effective, simultaneous treatment of a plurality of different or identical compositions of liquid media, each of which is situated in its own container. Specifically, the method may be advantageously used in the preparation of individual cremes with a sub-micron size dispersion phase.

Fig. 1

**Description**

This invention relates to fluid cavitation processing methods, and more specifically, to cavitation processing of fluids to have specific content of water or other liquid phase exceeding 30-35% of the total weight. Different fluid media placed in individual cells may be exposed to simultaneous processing.

The method and apparatus for simultaneous CA2025833 collagen processing dated 22.03.1991 that requires placing collagen vials (syringes) in a fluid-filled bath with its bottom ultrasonically vibrated at the frequency ranged within ~20 kHz to 3 MHz is known. Specific well-developed acoustic cavitation is produced within every cell exposed to processing. Drawback of this method is that fluid cannot be equally processed at different frequencies, since fluid level in the bath depends on cell volumes used for processing but not on the length of ultrasonic waves in fluid. Besides, a few frequencies only can define specific elastic properties of the bath bottom under vibration due to rather effective ultrasonic wave excitation in bath fluid. The first two/three natural vibration harmonics only, particularly those specified in the works /1-2/, may be referred to the above frequencies.

As applied to the cells placed in process fluid, cavitation processing efficiency is significantly reduced.

Another prior art describes the method for measuring biological tissue radiation parameters (patent JP 6207893 dated 26.07.1994).

The cells under measurement are placed in a vessel filled with process fluid.

Fluid is processed by ultrasonic acoustic wave excited from below through ultrasonic vibration tools immersed in the process fluid.

The cells where processed tissues are placed in are made of the materials which acoustic resistance is close to that of the process fluid. Thus, developed acoustic cavitation conditions are produced not only in the process fluid, but also inside the fluid cells where processed tissues are placed in. Similar to the prior art, the last one is disadvantaged by dependence of cavitation processing efficiency on level of the process fluid.

Should fluid level fail to be multiple of one fourth of acoustic wave length, a complex superposition of incident and reflected waves will be formed under surface reflection. As a result, optimal conditions of cavitation bubbling dynamics is inevitably changed and, in the large, cavitation effect is reduced.

Cosmetic emulsion production method (Pat RU 2427362 dated 08.09.2010) is the closest one applied. The acoustic cavitation conditions described in this method are formed under double resonance effect to occur inside the flowing mechanical vibratory system - a square channel on opposite sides of which in-phase sound vibration and a standing wave are generated at fundamental harmonic frequency, which in its turn forms a quasi-plane standing wave in moving processed medium at a gap between the channel walls, wherein width of the channel gap is a multiple of one fourth of the wavelength excited by the channel walls. As a result, a specific high-intensity acoustic wave is formed in the processed fluid at the same resonance frequency. Drawbacks of this method are that several cells to have different contents cannot be processed simultaneously, but efficiently processed at a single frequency rated against the channel wall first harmonic frequency and selected gap.

It is known that cavitation processes performed simultaneously at two different frequencies have their synergetic effect much larger than that produced serially at the both frequencies /3/.

It is an object of this invention to provide a technique for simultaneous processing of several fluid-filled cells to have several and single low-volume ingredient contents, as well as for processing under the simultaneous effect of several resonant acoustic waves.

This object is achieved by application of a square bath filled with process fluid where a standing acoustic wave is produced and reflected from the bath walls and bottom designed as elastic membranes to have self-resonant first-harmonic resonance frequency where the opposite square bath walls may have either equal or different first-harmonic frequencies, wherein length "**a**" and width "b" of the bath are selected as multiples of one fourth of the wavelength excited within process fluid by the lateral bath walls:

$$a=(k/4)*(c/f_i),$$

$$b=(k/4)*(c/f_i),$$

where

"**c**" is acoustic speed rated within process fluid, m/s;
"**f_i**" is bath lateral wall first-harmonic frequencies, Hz;
"k= 1,2,3..." is an integral number.

Height of process fluid level "h" is specified as a multiple of one fourth of the bath bottom-excited wavelength, wherein vibration frequencies "**f_i**" are rated by a cross-multiple factor "k".

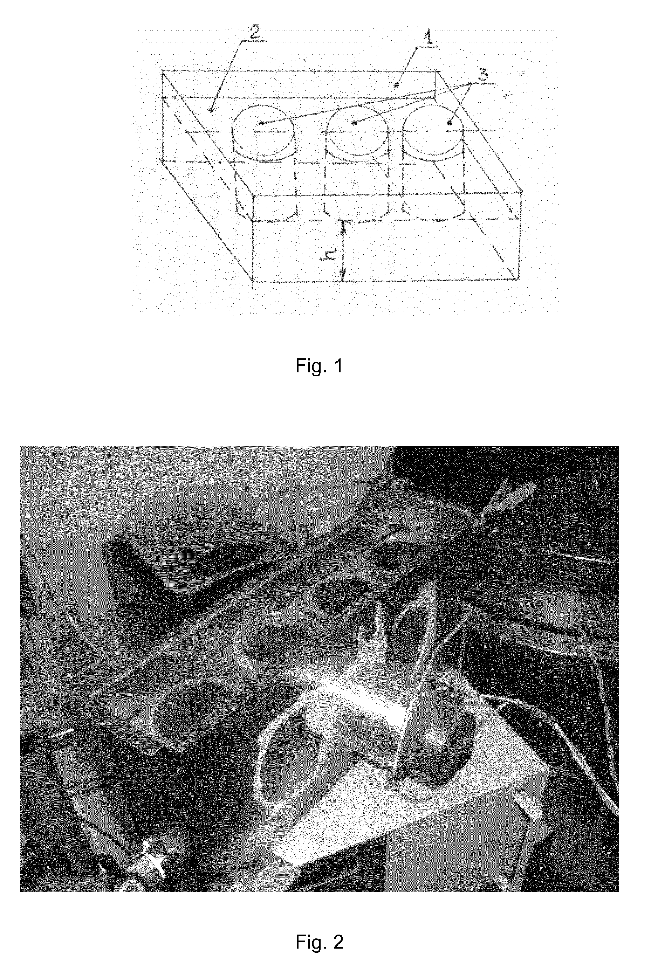

For implementation of this method, refer to Fig. 1. This figure illustrates the square bath 1 filled with process fluid 2 where the processed fluid media cells 3 are placed. Height of the process fluid level in the bath is rated by the value "h". Every square bath wall is designed as an elastic membrane. The highest cavitation effect and acoustic wave amplitude in the fluid is gained when this kind of a membrane generates first-harmonic forced vibration, thereby producing a first-harmonic standing wave on the membrane surface. Calculation of this frequency is known and does not present any difficulties /1-2/. The same concerns the square bath which lateral walls have equal frequencies. Figure 2 illustrates one of the techniques applied for simultaneous treatment of 4 fluid-filled cells at 24 Hz to have different and single ingredient contents. 100 ml plastic cups are used for preparation of 70-90 ml.

The rate of polyethylene acoustic resistance (density: 0.92-0.94 g/cm$^3$, longitudinal wave speed: $\sim$1900-1950 m/s) is approximately equal to the resistance of water selected for process fluid.

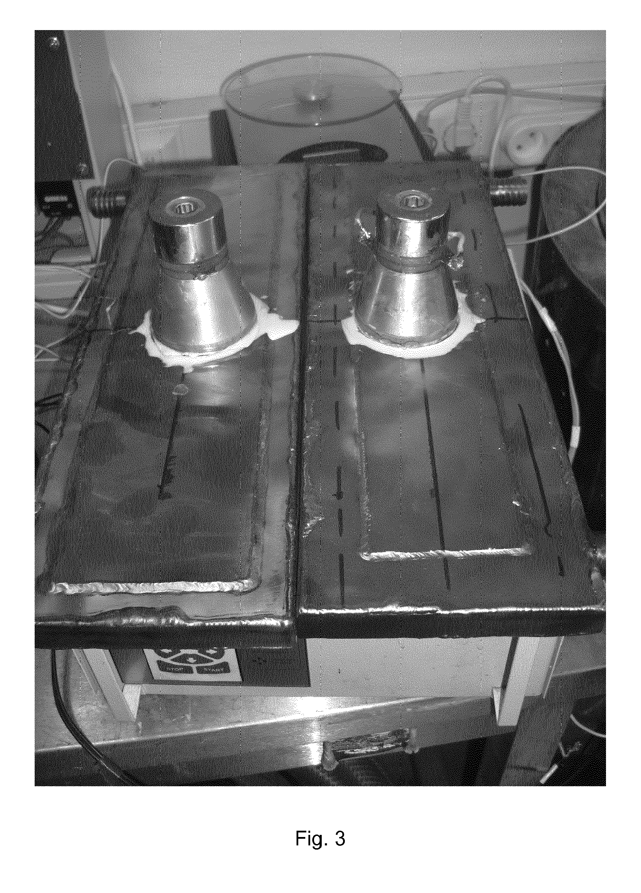

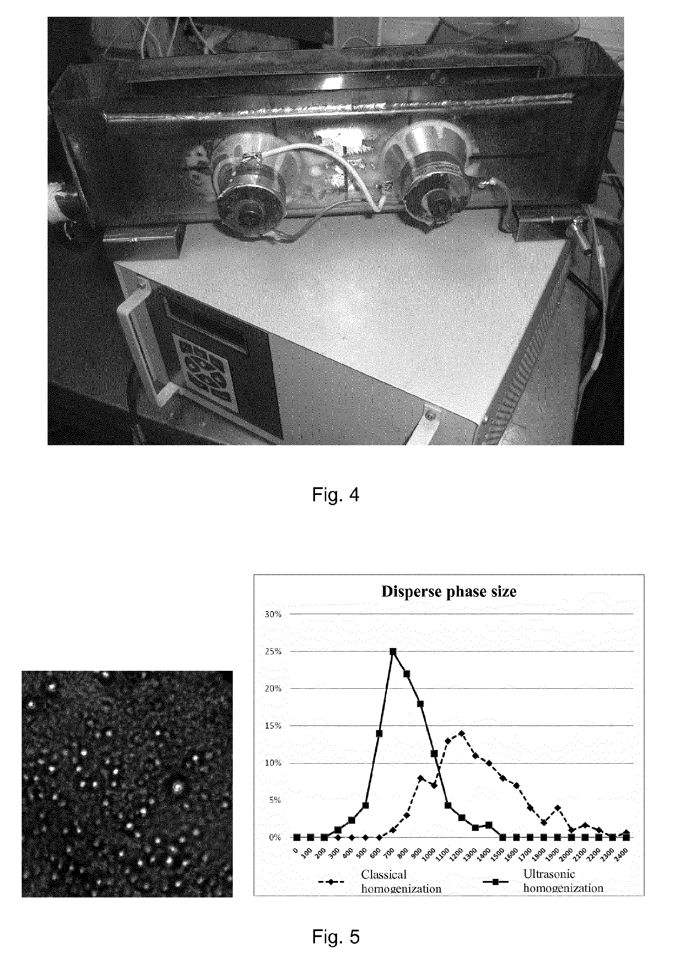

As concerns the opposite bath walls generating different frequencies, it is a matter of a problematic nature. In this case, a membrane may be reinforced with ribs and have the area less than that of the bath wall - for the technique of implementation, refer to Fig. 3 and Fig. 4.

For gaining the highest cavitation and collateral resonance effects a standing acoustic wave with selected frequencies "$f_i$" must be produced in a process fluid-filled cell. For these effects, internal bath dimensions must be multiple of one fourth of the wavelength excited in the fluid between lateral walls of the bath. Matching of standing wave nodes and loops is secondly conditioned by cross-multiplicity of frequencies "$f_i$". Height of process fluid level **"h"** is specified as a multiple of one fourth of the bath bottom-excited wavelength.

The total fluid double resonant effect produced by processing of fluid cells may be specifically utilized for preparation of small amounts of cosmetic emulsions intended for individual types of a customer skin. Specific cream structure and phospholipid-based (liquid crystals) microphotography obtained by means of polarizing microscope using Maruzen Pharm's formulation is demonstrated in Fig. 5. This kind of formulation applies to the luxury-class pricing segment. Quality of these structures may be verified against typical lipid membrane luminous effect fixed by a polirizing microscope only. It should be noted that crime structure is significnalty improved, dispersed phase is reduced by 2-3 times and homogeneity level is increased by 2 times with favorable organoletic crime properties produced after simultaneous processing of 4 cosmetics cells to have different active admixture composition, but identical emulsion base composition with specific resonsance standing wave excited in the process fluid.

Similar results have been obtained on processing suspensions, in particular chalk dental pastes, using SPLAT's formulation.

Hence, accomplishment of the object and commercial capabilites of this invention are duly acknowledged.

BIBLIOGRAPHY

1. Aramanovich I.G., Levin V.I., Mathematical Physics Equation, second edition, M, Science, 1969.
2. Technology Vibrations, reference manuals in 6 volumes under the editorship of Chelomey V.N., M, Mechanical Engineering, 1979
3. Margulis M.A. Fundamental Sonochemistry; Chemical Acoustic Field Reactions, - M: Higher School, 1984.

Claims

1. The method for ultrasonically and simultaneously induced cavitation processing of fluid media-filled cells that requires placing of cells in a bath filled with process fluid, wherein the fluid cell material exhibits specific acoustic resistance to be equal or close to that of the process fluid, sufficient acoustic wave amplitude is produced for specific process fluid well-developed acoustic cavitation to occur in process fluid and every cell exposed to processing, which is **characterized in that** the process fluid bath has a square form where acoustic standing wave is produced in process fluid and is reflected from the bath walls and bottom designed as elastic membranes to have self-resonant first harmonic frequency, wherein the square bath opposite walls may exhibit both equal and different first harmonic frequencies where length **"a"** and width **"b"** of the bath are selected as multiples of one fourth of the wavelength excited in process fluid by the lateral bath walls:

$$a=(k/4)*(c/f_i),$$

$$b=(k/4)*(c/f_i),$$

where

"c" is acoustic speed rated within process fluid, m/s;
"$f_i$" is bath lateral wall first-harmonic frequencies, Hz;
"k= 1,2,3..." is an integral number

Height of process fluid level **"h"** is specified as a multiple of one fourth of the bath bottom-excited wavelength, wherein vibration frequencies "$f_i$" are rated by a cross-multiple factor **"k".**

Fig. 1

Fig. 2

Fig. 3

Fig. 4

Fig. 5

| | INTERNATIONAL SEARCH REPORT | International application No. |
|---|---|---|
| | | PCT/RU 2012/000552 |

**A. CLASSIFICATION OF SUBJECT MATTER**

*B01F 3/00 (2006.01)*
*B01F 11/02 (2006.01)*
*A61K 8/06 (2006.01)*

According to International Patent Classification (IPC) or to both national classification and IPC

**B. FIELDS SEARCHED**

Minimum documentation searched (classification system followed by classification symbols)

C02F 1/34, 1/36, B01F 11/02, B08B 3/12, B01F 3/00-3/14, A61K 8/06, C08H 1/06

Documentation searched other than minimum documentation to the extent that such documents are included in the fields searched

Electronic data base consulted during the international search (name of data base and, where practicable, search terms used)

PatSearch (RUPTO internal), Esp@cenet, PAJ, USPTO

**C. DOCUMENTS CONSIDERED TO BE RELEVANT**

| Category* | Citation of document, with indication, where appropriate, of the relevant passages | Relevant to claim No. |
|---|---|---|
| A | EP 0555842 A1 (JAPAN SCIENCE INC) 18.08.1993 | 1 |
| A | RU 2427362 C1 (GETALOV ANDREI ALEKSANDROVICH) 27.08.201 | 1 |
| A | EP 0418979 A2 (ZOCCH1 MICHELE DR.) 27.03.1991 | 1 |

☐ Further documents are listed in the continuation of Box C.     ☐ See patent family annex.

| | |
|---|---|
| *   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 29 January 2013 (29.01.2013) | 14 February 2013 (14.02.2013) |

| Name and mailing address of the ISA/ | Authorized officer |
|---|---|
| | |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CA 2025833
- JP 6207893 B

- RU 2427362

**Non-patent literature cited in the description**

- **ARAMANOVICH I.G. ; LEVIN V.I.** Mathematical Physics Equation. 1969
- Technology Vibrations. **CHELOMEY V.N.** M, Mechanical Engineering. 1979

- **MARGULIS M.A.** Fundamental Sonochemistry; Chemical Acoustic Field Reactions. *M: Higher School,* 1984